# EUROPEAN PATENT APPLICATION

(11) **EP 4 091 635 A1**
(43) Date of publication of application: **23.11.2022**
(21) Application number: 21740676.8
(22) Date of filing: 14.01.2021
(51) Int. Cl.: A61K 48/00, A61P 25/28

(54) **PHARMACEUTICAL COMPOSITION FOR PREVENTING OR TREATING ALZHEIMER'S AND USE THEREOF**

(30) Priority: 14.01.2020 KR 20200004615
(71) Applicant: Toolgen Incorporated, Seoul 08501 (KR); Samsung Life Public Welfare Foundation, Seoul 04348 (KR)
(72) Inventor: SHIN, Eun Ji, Seoul 08501 (KR); LEE, Kang In, Seoul 08501 (KR); LEE, Nahee, Seoul 06351 (KR); CHANG, Jong Wook, Seoul 06351 (KR); LEE, Na Kyung, Seoul 06351 (KR); NA, Duk Lyul, Seoul 06351 (KR); LEE, Jae Young, Seoul 08501 (KR)
(74) Representative: dompatent von Kreisler Selting Werner - Partnerschaft von Patent- und Rechtsanwälten mbB
(86) International application number: PCT/KR2021/000550
(87) International publication number: WO 2021/145703

(57) **Abstract**

The present disclosure provides a pharmaceutical composition for treating Alzheimer's disease and use thereof, the pharmaceutical composition containing, as an active ingredient, stem cells (SC) having high adaptability under hypoxic conditions. Particularly, the present disclosure discloses a pharmaceutical composition for alleviating or treating the symptoms of Alzheimer's disease, the pharmaceutical composition containing, as an active ingredient, mesenchymal stem cells (MSC) which have high adaptability or viability in hypoxic environments and comprise one or more knock-out HIF1AN genes.

## Description

### Technical Field

The present application relates to a pharmaceutical composition for treating Alzheimer's disease and a use thereof, the pharmaceutical composition containing, as an active ingredient, stem cells (SCs) having high adaptability under hypoxic conditions.

### Background Art

Alzheimer's disease (AD) is a disease in which brain neurons (nerve cells) related to learning or memory die, resulting in loss of memory and gradual deterioration of thinking skills such as computational power, language ability, temporal and spatial comprehension, and judgment. Alzheimer's disease is one of the progressive neurologic disorders and accounts for 60% to 70% of the causes of dementia. Although the cause of Alzheimer's disease is not well known, apoptosis of neurons is commonly observed in the limbic pathway that controls memory. A likely cause of the progressive loss of cognitive function, which is a primary symptom of AD patients, appears to be due to abnormally accumulated amyloid beta (A β).

Recently, there has been an attempt to use stem cells for the treatment of Alzheimer's disease.

A stem cell drug is a drug used for the purpose of treating, diagnosing, and preventing disease. Unlike classical drug therapy, stem cell therapy is a new type of cell therapy in which stem cells collected from the human body are engineered in vitro and injected back into the patient. It can be used for prevention, symptom relief, or treatment of intractable diseases through regeneration, repair, and recovery of damaged tissues and cells. Stem cells are in the spotlight as a therapeutic agent because they have the ability to self-renew and can reduce the cumbersome treatment process or frequency, which is a problem with conventional gene therapy.

However, treatment using stem cells has problems such as immune rejection, erratic differentiation by migrating to non-targeted tissues, and the ability of stem cells to turn into cancer cells. In addition, low efficiency of stem cells stably engrafting and multiplying in a patient body is also a problem.

### [Documents of Related Art]

[Patent Document] (Patent Document 0001) COMPOSITION FOR CLEAVING A TARGET DNA COMPRISING A GUIDE RNA SPECIFIC FOR THE TARGET DNA AND CAS PROTEIN-ENCODING NUCLEIC ACID OR CAS PROTEIN, AND USE THEREOF (International Publication Number WO 2014/065596 A1).

[Non-patent Document][Non-patent Document 0001) Mahon PC, Hirota K, Semenza GL. FIH-1: a novel protein that interacts with HIF-1alpha and VHL to mediate repression of HIF-1 transcriptional activity. Genes Dev. 2001;15(20):2675-2686. doi:10.1101/gad.924501.

### Disclosure

### Technical Problem

An objective of the present disclosure is to provide a pharmaceutical composition for treating Alzheimer's disease, the pharmaceutical composition containing artificially manipulated stem cells as an active ingredient.

Another objective of the present disclosure is to provide a method for treating Alzheimer's disease using artificially manipulated stem cells and/or a pharmaceutical composition containing artificially manipulated stem cells as an active ingredient for treating Alzheimer's disease.

A further objective of the present disclosure is to provide a method of preparing a pharmaceutical composition for treating Alzheimer's disease, the pharmaceutical composition containing artificially manipulated stem cells as an active ingredient.

A yet further objective of the present disclosure is to provide a use of a pharmaceutical composition for treating Alzheimer's disease, the pharmaceutical composition containing artificially manipulated stem cells as an active ingredient.

### Technical Solution

The present disclosure provides a pharmaceutical composition containing an manipulated stem cell as an active ingredients for treating Alzheimer's disease, the manipulated stem cell comprising an engineered gene having an indel in a wild-type HIF1AN gene, in which a sequence of the engineered HIF1AN gene is different from the original sequence of the wild-type HIF1AN gene, an expression level of FIH-1 in the manipulated stem cell is lower than that in a wild-type cell, and HIFα exhibits a higher expression level in the manipulated stem cell than in a wild-type cell.

In one embodiment, the indel of the engineered HIF1AN gene is located in an exon 1 region of the wild-type HIF1AN gene.

In one embodiment, the artificially manipulated stem cell may be an artificially engineered mesenchymal stem cell.

In one embodiment, the pharmaceutical composition may be a formulation for administration to a brain tissue.

In one embodiment, the brain tissue may be a hippocampus or a caudate putamen (CPu).

Present disclosure provides a pharmaceutical composition containing an manipulated stem cell as an active ingredients for treating Alzheimer's disease, the manipulated stem cell including an engineered HIF1AN gene, in which the engineered HIF1AN gene does not comprise one or more sequences selected from SEQ ID NOs:1 to 9, FIH-1 exhibits a lower expression level in the manipulated stem cell than in the wild-type cell, and HIFα exhibits a higher expression level in the manipulated stem cell than the wild-type cell.

The present disclosure provides a method for treating Alzheimer's disease, the method including: administering a pharmaceutical composition containing manipulated stem cells as an active ingredient into a subject, in which the manipulated stem cell includes an engineered gene having an indel in a wild-type HIF1AN gene, a sequence of the engineered HIF1AN gene is different from the original sequence of the wild-type HIF1AN gene, FIH-1 exhibits a lower expression level in the manipulated stem cell than in the wild-type cell, and HIFα exhibits a higher expression level in the manipulated stem cell than the wild-type cell.

In one embodiment, the administration of the pharmaceutical composition may be a direct injection of the pharmaceutical composition into the brain tissue of a subject.

In one embodiment, the brain tissue may be a hippocampus or a caudate putamen (CPu).

In one embodiment, the indel of the engineered HIF1AN gene is located in an exon 1 region of the wild-type HIF1AN gene.

In one embodiment, the artificially manipulated stem cell may be an artificially engineered mesenchymal stem cell.

The present disclosure provides a method for treating Alzheimer's disease, the method including: administering a pharmaceutical composition comprising an manipulated stem cell as an active ingredients into a subject, wherein the manipulated stem cell comprises an engineered HIF1AN gene, wherein the engineered HIF1AN gene does not comprise one or more sequences selected from SEQ ID NOs:1 to 9, wherein FIH-1 exhibits a lower expression level in the manipulated stem cell than in the wild-type cell, and wherein HIFα exhibits a higher expression level in the manipulated stem cell than the wild-type cell.

The present disclosure provides a use of an manipulated stem cell for treating the Alzheimer's disease. The use may include administering a pharmaceutical composition containing artificially engineered stems cell as an active ingredients into a subject, the artificially manipulated stem cell including an engineered gene having an indel in a wild-type HIF1AN gene, in which a sequence of the engineered HIF1AN gene is different from the original sequence of the wild-type HIF1AN gene, an expression level of FIH-1 in the manipulated stem cell is lower than that in a wild-type cell, and HIFα exhibits a higher expression level in the manipulated stem cell than in a wild-type cell.

In one embodiment, the administration of the pharmaceutical composition may be a direct injection of the pharmaceutical composition into the brain tissue of a subject.

In one embodiment, the brain tissue may be a hippocampus or a caudate putamen (CPu).

In one embodiment, the indel of the engineered HIF1AN HIF1AN gene may be located in an exon 1 region of the wild-type HIF1AN gene.

In one embodiment, the artificially manipulated stem cell may be an artificially engineered mesenchymal stem cell.

Present disclosure provides a use of a pharmaceutical composition containing an manipulated stem cell as an active ingredients for treating Alzheimer's disease, the manipulated stem cell including an engineered HIF1AN gene, in which the engineered HIF1AN gene does not include one or more sequences selected from SEQ ID NOs:1 to 9, FIH-1 exhibits a lower expression level in the manipulated stem cell than in a wild-type cell, and HIFα exhibits a higher expression level in the manipulated stem cell than in a wild-type cell.

The present disclosure provides a use of an manipulated stem cell as a medicament for treatment of Alzheimer's disease, the manipulated stem cell including an engineered gene having an indel in a wild-type HIF1AN gene, in which a sequence of the engineered HIF1AN gene is different from the original sequence of the wild-type HIF1AN gene, FIH-1 exhibits a lower expression level in the manipulated stem cell than in a wild-type cell, and HIFα exhibits a higher expression level in the manipulated stem cell than in a wild-type cell.

In one embodiment, the medicament for treatment of Alzheimer's disease may be a formulation for administration to a brain tissue.

In one embodiment, the brain tissue may be a hippocampus or a caudate putamen (CPu).

In one embodiment, the indel of the engineered HIF1AN gene may be located in an exon 1 region of the wild-type HIF1AN gene.

In one embodiment, the artificially manipulated stem cell may be an artificially engineered mesenchymal stem cell.

The present disclosure provides a use of an manipulated stem cell for the manufacture of a medicament for treatment of Alzheimer's disease. The use includes administering a pharmaceutical composition containing an manipulated stem cell as an active ingredient into a subject. The manipulated stem cell includes an engineered HIF1AN gene, the engineered HIF1AN gene does not comprise one or more sequences selected from SEQ ID NOs:1 to 9, FIH-1 exhibits a lower expression level in the manipulated stem cell than in a wild-type cell, and HIFα exhibits a higher expression level in the manipulated stem cell than in a wild-type cell

### Advantageous Effects

The present disclosure can provide a stable and effective medicament or therapeutic pharmaceutical composition for treating Alzheimer's disease and also can provide an Alzheimer's disease treatment method using the same.

The present disclosure can provide a pharmaceutical composition for alleviating or treating the symptoms of Alzheimer's disease, the pharmaceutical composition containing, as an active ingredient, mesenchymal stem cells (MSC) that have high adaptability or viability in hypoxic environments and which include one or more knock-out HIF1AN genes.

### Description of Drawings

FIG. 1 is experimental data for checking whether gene engineering is performed after treating a hHIFlAN gene with a gene engineering composition targeting a portion of the nucleotide sequence of the hHIFlAN gene. Specifically, the indel generation rate was checked with change in guide nucleic acid.
FIGS. 2 show cells suitable for experiments obtained by treating MSC with a hHIFlAN gene-engineering composition and selecting the treated MSC through deep sequencing.

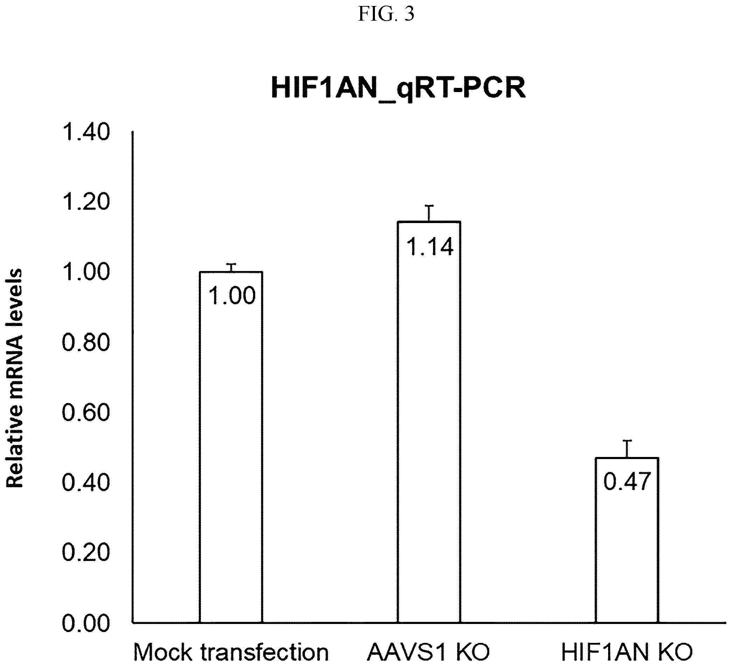

FIG. 3 is comparison data showing expression levels of mRNA transcribed from an HF1AN gene levels in the cells selected through deep sequencing in FIG. 2, in which the mRNA expression levels are compared through qRT-PCR.
FIG. 4 is a graph showing a result of an experiment of measuring the change in the proliferative capacity of MSCs by inducing reactive oxidative stress (ROS), which often occurs under hypoxic conditions, with hydrogen peroxide (H₂O₂), in which the graph shows the number of survived cells counted through CCK-8 assay. In this data, SFM stands for serum free medium.

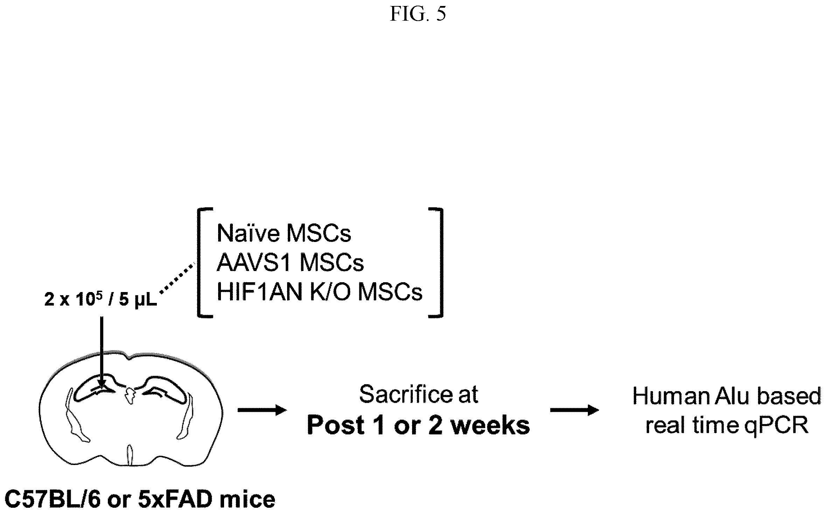

FIG. 5 is a schematic diagram of an in-vivo experiment targeting C57BL/6 and 5xFAD mice.
FIG. 6 is a graph showing the experimental results of measuring the viability of MSCs in which the HIF1AN gene is knocked out by qPCR in an in-vivo experiment targeting C57BL/6 mice.
FIG. 7 is a graph showing the experimental results of measuring the viability of MSCs in which the HIF1AN gene is knocked out by qPCR in an in-vivo experiment targeting 5xFAD mice.
FIG. 8 is a graph for determining whether the expression of soluble Aβ was reduced in the group administered with HIF1AN K/O stem cells compared to the control group (naive/AAVS1) in an in-vivo experiment targeting 5xFAD mice.

### Best Mode

Hereinafter, with reference to the accompanying drawings, the invention will be described in more detail through specific embodiments and examples. It should be noted that the accompanying drawings include some, but not all, embodiments of the invention. The details of the invention disclosed in the present specification may be embodied in various forms, and are not limited to the specific embodiments described herein. These embodiments are considered to be provided in order to satisfy the statutory requirements applicable herein. Those skilled in the art to which the invention disclosed herein pertains will come up with many modifications and other embodiments of the subject matter disclosed herein. Accordingly, it is to be understood that the subject matter disclosed herein is not limited to the specific embodiments described herein, and that modifications and other embodiments thereof also fall within the scope of the claims.

### Definition of Terms

### About

As used herein, the term "about" refers to a degree close to a certain quantity, and it refers to an amount, level, value, number, frequency, percent, dimension, size, amount, weight, or length that varies by to the extent of 30%, 25%, 20%, 15%, 10%, 9%, 8%, 7%, 6%, 5%, 4%, 3%, 2%, or 1% with respect to a reference amount, level, value, number, frequency, percentage, dimension, size, amount, weight, or length.

### Meaning of A, T, C, G, and U

As used herein, the symbols A, T, C, G and U are interpreted as meanings understood by those of ordinary skill in the art. Each of these symbols may be properly interpreted as a base, a nucleoside, or a nucleotide on DNA or RNA according to context and technology. For example, when each of the symbols means a base, the symbols A, T, C, G and U can be interpreted as adenine (A), thymine (T), cytosine (C), guanine (G), or uracil (U), respectively. When each of the symbols means a nucleoside, the symbols A, T, C, G and U can be interpreted as adenosine (A), thymine (T), cytidine (C), guanosine (G) or uridine (U), respectively. When meaning a nucleotide in the sequence, the symbols A, T, C, G and U denote nucleotides including the nucleosides, respectively.

### Engineered

As used herein, the term "engineered" is a term used to distinguish a certain substance, molecule, etc. from a substance, molecule, etc. having a composition already existing in nature, and the term "engineered" means that artificial modifications are applied to substances, molecules, etc. For example, in the case of "engineered gene", it refers to a gene in which an artificial change has been made to the composition of a gene existing in nature. In addition, the term include all meanings recognizable by those skilled in the art and may be appropriately interpreted according to the context.

### Wild-type

The term "Wild-type" means that a gene comprising a naturally occurring nucleotide sequence and a protein expressed from the gene have normal functional properties. Wild-type genes are most frequently observed in the population. When the term "wild-type" is used herein in contrast to an artificially manipulated (engineered) gene and/or cell, it means a gene that is the same kind as a corresponding engineered gene and includes a "non-artificially engineered" and naturally occurring nucleotide sequence, and a cell including the gene. In addition, the term include all meanings recognizable by those skilled in the art and may be appropriately interpreted according to the context.

### Knock-out

As used herein, the expression "knock-out" or "knockout gene" means that a protein expressed by a wild-type gene cannot be produced through transcription and/or translation. For example, a cell containing a knocked-out gene A may not express mRNA and/or protein expressed by a wild-type gene A. A cell including a knocked-out gene A may be cell in which only one of the genes A present in the cell is knocked out, or two or more genes of the genes A are knocked out. In addition, the term include all meanings recognizable by those skilled in the art and may be appropriately interpreted according to the context.

### Knock-down

As used herein, the term "knock-down" or "knock-down gene" means expressing a substance in a lower amount than a wild-type gene. For example, a cell including a knocked-down gene A may express a smaller amount of mRNA than the mRNA expressed by a wild-type gene A. A cell with a knocked-out gene A may be a cell in which only one of the genes A present in the cell is knocked down, or two or more genes of the genes A are knocked down. In addition, the term include all meanings recognizable by those skilled in the art and may be appropriately interpreted according to the context.

### Expression Level

As used herein, the term "expression level" refers to how well expression products, for example, mRNA and/or proteins are produced from a particular gene. In general, when the expression level of a specific gene in a cell is high, the amount of mRNA and/or a specific protein produced from the specific gene contained in the cell, or the concentration of the expression products in the cytoplasm is high. On the contrary, when the expression level of a specific gene in a cell is low, the amount of mRNA and/or a specific protein produced from the specific gene contained in the cell, or the concentration of the expression products in the cytoplasm is low. In the present disclosure, when encountering the expression "expression level is high" or "expression level is low", the comparison target may be interpreted as a homogeneous, wild-type cell, unless the comparison target is otherwise specified. In addition, quantitative indicators that serve as comparative references should be interpreted as most appropriate in context. For example, an absolute amount, a relative amount, and/or a concentration of each cell may be a comparison criterion, but is not limited thereto. For example, when it is expressed that the expression level of the gene a in a first cell is higher than the expression level of the gene a in a second cell, the expression product (mRNA, protein, etc.) of the gene a in the first cell is larger in absolute amount, relative amount, and/or concentration than the expression product of the gene a in the second cell. In addition, the term include all meanings recognizable by those skilled in the art and may be appropriately interpreted according to the context.

### Background Art - Alzheimer's Disease

Alzheimer's disease (AD) is a disease in which brain neurons (nerve cells) related to learning or memory dies, resulting in loss of memory and gradual deterioration of thinking skills such as computational power, language ability, temporal and spatial comprehension, and judgment. Alzheimer's disease is one of the progressive neurologic disorders and accounts for 60% to 70% of the causes of dementia. Although the cause of Alzheimer's disease is not well known, apoptosis of neurons is commonly observed in the limbic pathway that controls memory. A likely cause of the progressive loss of cognitive function, which is a primary symptom of AD patients, appears to be due to abnormally accumulated amyloid beta (Aβ).

### Background Art - Cell Therapeutics

### Concept of Cell Therapy

Recently, a method of using cells as a therapeutic agent for the purpose of treating, diagnosing, and preventing a disease has been attracting attention. In general, cells are administered directly to the relevant site for the treatment of diseases or regeneration of damaged tissues or organs. As the cells to be administered, cells capable of expressing a therapeutic substance or cells having intact functions are used. Cells administered to a specific site can achieve the therapeutic or regenerative purpose by surviving for a long period of time at the administered site and performing a normal function.

### Stem Cell Drug

A stem cell drug is a drug used for the purpose of treating, diagnosing, and preventing disease. Unlike classical drug therapy, stem cell therapy is a new type of cell therapy in which stem cells collected from the human body are engineered in vitro and injected back into the patient. The stem cell drug can be used for prevention, symptom relief, or treatment of intractable diseases through regeneration, repair, and recovery of damaged tissues and cells. Stem cells are in the spotlight as a therapeutic agent because they have the ability to self-renew and can reduce the cumbersome treatment process or frequency, which is a problem with conventional gene therapy.

However, treatment using stem cells has problems such as immune rejection, erratic differentiation by migrating to non-targeted tissues, and the ability of stem cells to turn into cancer cells. In addition, low efficiency of stem cells stably engrafting and multiplying in a patient body is also a problem.

### Background Art - HIF1 Protein and Factors Inhibiting Activity Thereof

### HIF1 Protein

It is known that cells placed in a hypoxic environment express a large amount of a hypoxia-inducible factor 1 (HIF1) protein in order to survive. HIF1 protein is a gene transcriptional regulator involved in angiogenesis (vascularization) and energy metabolism in a hypoxic environment, thereby playing a role in creating an environment in which cells can survive. HIF1 has HIF1α and HIF1β as subunits thereof. HIF1α and HIF1 β are encoded by HIF1A and HIF1B genes, respectively. Therefore, in order for cells to survive in a hypoxic environment, the expression level of the HIF1 protein or the expression levels of the subunits of the HIF1 protein must be high, or the activity thereof must be maintained high.

### FIH1 - Factor Inhibiting Activity of HIF1 Protein

FIH-1 is a protein encoded by the HIF1AN gene and is a protein involved in the post-translational control of HIF1α. More specifically, FIH-1 is an asparaginyl hydroxylase. FIH-1 plays a role in reducing HIF1 activity by interfering with the interaction between HIF1α and p300/CBP, which is a nuclear receptor activator. Since FIH-1 even works under severe hypoxia, it corresponds to a protein directly related to cell survival in a hypoxic environment.

### Limitations of Existing Arts

To date, studies on AD have mainly focused on development of agents for preventing and treating AD using A β production inhibitors such as secretase inhibitors or neurotoxic inhibitors such as antioxidants. At the present, the following AD-related therapeutic agents are being developed: ABT-418, which is a nicotin receptor agonist; muscarin receptor agonist, Xanomeline, YM-796; metal chelators such as desferrioxamine and clioquinol; amyloid beta inhibitors such as Aducanumab, Bapineuzumab, CAD106, Gantenerumab, and Solanezumab; β-site amyloid precursor protein cleaving enzyme (BACE) inhibitors such as AZD3293, E2609, JNJ-54861911, and MK-8931; a tau protein inhibitor, TRx-023; intranasal insulins; Pioglitazone, which is a PPAR-γ receptor agonist; and Nilvadipine, which is a dihydropyridine calcium channel blocker. However, most of the substances are insignificant in efficacy to the extent of alleviating some pathological symptoms or slowing the progression of symptoms or are difficult to use due to their toxicity. Therefore, there is an urgent need for development of a stable and effective therapeutic agent for AD.

Recently, there has been an attempt to introduce the aforementioned cell therapeutic agent, specifically, a stem cell therapeutic agent for prevention and treatment of AD. However, in the case of selecting and multiplying cells for treatment or the like in vitro and then administering the cells into a living body, there is a problem in that the administered cells quickly die due to the in vivo environment. A typical reason for cell death (apoptosis) is that i) the in vitro environment in which the cells are cultured is different from the in vivo environment, or ii) the administered site in the body is maintained in a hypoxic environment. Therefore, in order to apply a stem cell therapeutic agent to AD prevention and treatment, it is required to solve the problem that the administered cells die in a hypoxic environment.

### Pharmaceutical Composition for Treating Alzheimer's Disease

### Overview

The present disclosure discloses a pharmaceutical composition for treating Alzheimer's disease, the pharmaceutical composition containing artificially engineered stem cells as an active ingredient. The pharmaceutical composition includes artificially engineered stem cells obtained by engineering a wild-type HIF1AN gene. The artificially engineered stem cells are characterized in that they exhibit a lower expression level of mRNA and/or FIH-1 protein transcribed by the HIF1AN gene than wild-type cells, whereby the intracellular HIFα expression level and/or activity level of the engineered stem cells is higher than that of wild-type cells. The pharmaceutical composition for treating Alzheimer's disease disclosed herein may include a pharmaceutically acceptable carrier in addition to the artificially engineered stem cells, and may be formulated into various dosage forms or preparations.

In one embodiment, the pharmaceutical composition for treating Alzheimer's disease may include artificially engineered stem cells containing the engineered HIF1AN gene as an active ingredient. In one embodiment, the engineered HIF1AN gene may be a gene having an indel in the corresponding HIF1AN wild-type gene. In this case, the sequence of the engineered HIF1AN gene may be different from the sequence of the wild-type HIF1AN gene. In one embodiment, the expression level of FIH-1 in the engineered stem cells may be higher than that in the wild-type cells. In one embodiment, the HIF1α expression level and or the HIF1α activity level in the engineered cell may be lower than that in the wild-type cells.

### Artificially Engineered Stem Cells 1 - Cell Types

In one embodiment, the artificially engineered stem cells disclosed herein are embryonic stem cells, neural stem cells, mesenchymal stem cells, adult stem cells which have improved viability in a hypoxic environment. For example, the stem cells may be tissue-derived stem cells derived from adipose, uterus, bone marrow, liver, muscle, placenta, nerve, umbilical cord blood, or skin (epithelium). Alternatively, the stem cells may be induced pluripotent stem cells (iPSCs). In one embodiment, the artificially engineered stem cells may be mesenchymal stem cells (MSCs). In one embodiment, the artificially engineered stem cells may be mesenchymal stem cells derived from human Wharton's jelly. In one embodiment, the artificially engineered stem cells may be mesenchymal stem cells that are negative for CD34, and/or CD45 markers. In one embodiment, the artificially engineered stem cells may be mesenchymal stem cells that are positive for CD73, and/or CD90 markers.

### Artificially Engineered Stem Cells 2 - Including Engineered HIF1AN

In the artificially engineered stem cells, it is characterized in that a wild-type HIF1AN gene expressing a substance that inhibits transcription, expression, and/or activity of a HIF1 protein is artificially edited. In other words, the cell includes a HIF1AN gene that is engineered on a genomic sequence thereof, and has a reduced ability to express a substance that inhibits the transcription, expression, and/or activity of the HIF1 protein.

In one embodiment, the cell may include an engineered HIF1AN. In this case, the HIF1AN gene may be a human gene, but is not limited thereto. In this case, the sequence of the engineered HIF1AN gene may be different from the sequence of the wild-type HIF1AN gene.

### Artificially Engineered Stem Cells 3 - Characteristics of Engineered HIF1AN

The engineered HIF1AN gene is characterized in that it has a sequence different from the sequence of the corresponding wild-type gene. The engineered HIF1AN gene may not be able to express the substance that can be expressed by a non-engineered wild-type HIF1AN gene. In the engineered HIF1AN gene may less express the substance expressed by a wild-type HIF1AN gene. In this case, the substance expressed by the wild-type HIF1AN gene may be FIH-1 protein.

In one embodiment, the engineered HIF1AN gene may be a mutation form of the wild-type HIF1AN gene. In this case, the mutation may not be a mutation that occurs in nature but may be an artificially generated mutation.

In one embodiment, the engineered HIF1AN gene may be a knock-out form of the corresponding wild-type HIF1AN gene.

In one embodiment, the engineered HIF1AN gene may be a knock-down form of the corresponding HIF1AN wild-type gene.

In one embodiment, the engineered HIF1AN gene may have one or more nucleotides inserted, deleted, substituted, and/or inverted compared to the corresponding wild-type HIF1AN gene.

In one embodiment, the engineered HIF1AN gene may be a gene having an indel in the corresponding wild-type HIF1AN gene. In this case, the indel is generated by a non-homologous end joining (NHEJ) mechanism in the cell, and one or more nucleotides in the wild-type gene may be inserted, deleted, and/or substituted by the NHEJ mechanism. In this case, the expression "including an indel in a gene" refers to an insertion, deletion, and/or substitution of one or more nucleotides in a wild-type gene, and/or a result thereof.

For example, an engineered A gene having an indel in a wild-type A gene may have one or more nucleotides inserted at any position in the wild-type A gene. Alternatively, for example, an engineered A gene having an indel in a wild-type A gene may have one or more nucleotides deleted at any position in the wild-type A gene. Further alternatively, for example, an engineered A gene having an indel in a wild-type A gene may be a form in which one or more nucleotides at any position in the wild-type A gene are substituted.

### Artificially Engineered Stem Cells 4 - Engineered HIF1AN Region

The artificially engineered stem cell is characterized in that it is a form in which a wild-type HIF1AN gene expressing a substance that inhibits transcription, expression, and/or activity of HIF1 protein is artificially edited. This means that one or more regions within the wild-type HIF1AN gene are engineered. In this case the engineered region in the wild-type HIF1AN gene is not particularly limited as long as the objectives mentioned above can be achieved.

In one embodiment, the artificially engineered stem cell may a form in which an exon region, an intron region, and/or a regulatory region in a wild-type HIF1AN gene is edited.

In one embodiment, the artificially engineered stem cell may include an engineered HIF1AN gene that is a form in which one or more regions selected from the group consisting of exon 1, exon 2, exon 3, exon 4, exon 5, ..., and exon N in a wild-type HIF1AN gene are artificially edited. In this case, N is an arbitrary integer. In one embodiment, the artificially engineered stem cell may include an engineered HIF1AN gene that is a form in which one or more regions selected from the group consisting of intron 1, intron 2, intron 3, intron 4, intron 5, ..., and intron M in a wild-type HIF1AN gene are artificially edited. In this case, M is an arbitrary integer.

In one embodiment, the cell may include an engineered HIF1AN gene that is a form in which exon 1 in a wild-type HIF1AN gene is artificially engineered.

### Artificially Engineered Stem Cells 5 - Example of Sequence of Engineered HIF1AN

In one embodiment, the artificially engineered stem cell provided by the present disclosure may have a form in which one or more sequences selected from SEQ ID NOs: 1 to 9 in an HIF1AN gene are artificially edited. In one embodiment, the artificially engineered stem cell may include an engineered HIF1AN gene, and the sequence of the engineered HIF1AN gene may not include one or more sequences selected from SEQ ID NOs: 1 to 9. In other words, a sequence identical to one or more sequences selected from SEQ ID NOs: 1 to 9 among the sequences of the engineered HIF1AN gene may not exist.

### Artificially Engineered Stem Cells 6 - Result of Expression of Engineered HIF1AN

The artificially engineered cells disclosed in the present disclosure are characterized in that a wild-type HIF1AN gene expressing a substance that inhibits the expression of HIF1 protein is artificially engineered, so that the wild-type HIF1AN gene cannot express the substance or expresses a smaller amount of the substance. Accordingly, the cells exhibit a lower expression level for a HIF1 expression negative regulator than the corresponding wild-type cell. Accordingly, the substance that inhibits the expression of the HIF1 protein (i.e., HIF1 expression negative regulator) may be FIH-1 protein.

In one embodiment, the artificially engineered stem cell includes an engineered HIF1AN gene having an indel in a wild-type HIF1AN gene. The sequence of mRNA expressed in the engineered HIF1AN gene may be different from the sequence of mRNA expressed in the wild-type HIF1AN gene. In one embodiment, the engineered stem cell is characterized in that the engineered stem cell includes an engineered HIF1AN gene having an indel in a wild-type HIFlAn gene. The mRNA sequence expressed in the engineered HIF1AN gene may be the same as the mRNA sequence expressed in the wild-type HIFlAn gene. In this case, the expression level of mRNA expressed in the engineered HIF1AN gene in the artificially engineered stem cell may be lower than the expression level of mRNA expressed in the wild-type HIF1AN gene in the wild-type cell.

In one embodiment, the artificially engineered stem cells may be cells in which the amount of mRNA expressed from the HIF1AN gene is reduced. In one embodiment, the expression level of mRNA expressed from the HIF1AN gene in the engineered stem cells may be lower than that in wild-type cells.

In one embodiment, when the artificially engineered stem cell includes an engineered HIF1AN gene having an indel in a HIF1AN gene, the concentration of mRNA and/or FIH-1 expressed from the HIF1AN gene included in the artificially engineered stem cell may be lower than the concentration in a wild-type cell. In one embodiment, when the artificially engineered stem cell includes an engineered HIF1AN gene having an indel in a HIF1AN gene, the expression level of mRNA and/or FIH-1 expressed from the HIF1AN gene included in the cell may be lower than the expression level in a wild-type cell.

### Artificially Engineered Stem Cells 7 - Example of Quantitative Indicator Related to Expression Product of Engineered HIF1AN

In one embodiment, the artificially engineered stem cell may exhibit an mRNA expression level that is about 0.9 times, about 0.85 times, about 0.8 times, about 0.75 times, about 0.7 times, about 0.65 times, about 0.6 times, about 0.55 times, about 0.5 times, about 0.45 times, about 0.4 times, about 0.35 times, about 0.3 times, about 0.25 times, about 0.2 times, about 0.15 times, about 0.10 times, about 0.05 times, about 0.04 times, about 0.03 times, about 0.02 times, or about 0.01 or less times the mRNA expression level of a wild-type cell. In one embodiment, the artificially engineered stem cell may exhibit an mRNA expression level within the range of two numerical values selected in the previous sentence compared to a wild-type cell. For example, the artificially engineered stem cell may exhibit an mRNA expression level in the range of 0.6 times to 0.7 times compared to a wild-type cell.

In one embodiment, the artificially engineered stem cell may exhibit a FIH-1 expression level that is about 0.9 times, about 0.85 times, about 0.8 times, about 0.75 times, about 0.7 times, about 0.65 times, about 0.6 times, about 0.55 times, about 0.5 times, about 0.45 times, about 0.4 times, about 0.35 times, about 0.3 times, about 0.25 times, about 0.2 times, about 0.15 times, about 0.10 times, about 0.05 times, about 0.04 times, about 0.03 times, about 0.02 times, or about 0.01 or less times the FIH-1 expression level of a wild-type cell. In one embodiment, the artificially engineered stem cell may exhibit an FIH-1 expression level within the range of two numerical values selected in the previous sentence compared to a wild-type cell. For example, the artificially engineered stem cell may exhibit an FIH-1 expression level in the range of 0.6 times to 0.7 times compared to a wild-type cell.

### Artificially Engineered Stem Cells 8 - HIFα Protein Expression Level

The cells having high adaptability to a hypoxic environment disclosed herein have a high intracellular HIF1α expression level or exhibits a high HIF1α activity.

In one embodiment, the cells having a high adaptability to a hypoxic environment may have a higher HIF1α expression level than wild-type cells. In this case, the HIF1α expression level may be the amount and/or concentration of HIF1α in the cell, but is not limited thereto.

In one embodiment, the cells having a high adaptability to a hypoxic environment may exhibit a higher HIF1α activity than wild-type cells.

### Artificially Engineered Stem Cells 9 - Example of Quantitative Indicator Related to HIFα Protein Expression Level

In one embodiment, the artificially engineered stem cell may exhibit a HIF1α expression level that is about 1.1 times, about 1.2 times, about 1.3 times, about 1.4 times, about 1.5 times, about 1.6 times, about 1.7 times, about 1.8 times, about 1.9 times, about 2.0 times, about 2.1 times, about 2.2 times, about 2.3 times, about 2.4 times, about 2.5 times, about 2.6 times, about 2.7 times, about 2.8 times, about 2.9 times, or about 3.0 or more times the HIF1α expression level of a wild-type cell. In one embodiment, the artificially engineered stem cell may exhibit an HIF1α expression level within the range of two numerical values selected in the previous sentence compared to a wild-type cell. For example, the artificially engineered stem cell may exhibit an HIF1α expression level in the range of 1.1 times to 1.5 times compared to a wild-type cell.

In one embodiment, the artificially engineered stem cell may exhibit a HIF1α activity level that is about 1.1 times, about 1.2 times, about 1.3 times, about 1.4 times, about 1.5 times, about 1.6 times, about 1.7 times, about 1.8 times, about 1.9 times, about 2.0 times, about 2.1 times, about 2.2 times, about 2.3 times, about 2.4 times, about 2.5 times, about 2.6 times, about 2.7 times, about 2.8 times, about 2.9 times, or about 3.0 or more times the HIF1α activity level of a wild-type cell. In one embodiment, the artificially engineered stem cell may exhibit an HIF1α activity level within the range of two numerical values selected in the previous sentence compared to a wild-type cell. For example, the artificially engineered stem cell may exhibit an HIF1α activity level in the range of 1.1 times to 1.5 times compared to a wild-type cell.

### Artificially Engineered Stem Cells 10 - Cell Culture Environment

In one embodiment, the artificially engineered stem cells may be cultured in a hypoxic environment. Specifically, the artificially engineered stem cells may be pre-conditioned an in vitro or in vivo hypoxic environment. The pre-conditioning in a hypoxic environment may improve the adaptability or viability of the stem cells in a hypoxic environment.

### Characteristic 1 of Artificially Engineered Stem Cell - High Viability in Hypoxic Environment

The artificially engineered stem cells disclosed herein are characterized in that they exhibit improved adaptability or viability in a hypoxic environment. In this case, the term "hypoxic environment" means a gaseous environment containing a low concentration of oxygen (O₂). As a result, when the artificially engineered stem cells are injected into the body of a living organism (for example, a human), the cells show improved adaptability or viability.

In one embodiment, the artificially engineered stem cells disclosed herein are characterized in that they survive for a longer period of time than wild-type cells in a hypoxic environment.

### Characteristic 2 of Artificially Engineered Stem Cells-Example of Quantitative Indicator of High Survival Rate in Hypoxic Environment

In one embodiment, the hypoxic environment may mean a gaseous environment in which the concentration of O₂ is about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, about 20%, or about 21% or less. In one embodiment, the hypoxic environment may be an artificially created environment that mimics the hypoxic environment. In one embodiment, the hypoxic environment may be an environment in which reactive oxidative stress (ROS) commonly occurring in the hypoxic environment is artificially induced with H₂O₂.

In one embodiment, when one or more wild-type cells and one or more artificially engineered stem cells disclosed herein are placed in the same hypoxic environment for a predetermined period of time, the number of viable cells of the artificially engineered stem cells may be about 1.01 times, about 1.02 times, about 1.03 times, about 1.04 times, about 1.05 times, about 1.06 times, about 1.07 times, about 1.08 times, about 1.09 times, about 1.1 times, about 1.15 times, about 1.2 times, about 1.25 times. times, about 1.3 times, about 1.35 times, about 1.4 times, about 1.45 times, about 1.5 times, about 1.55 times, about 1.6 times, about 1.65 times, about 1.7 times, about 1.75 times, about 1.8 times, about 1.85 times, about 1.9 times, about 1.95 times, about 2.0 times, about 2.1 times, about 2.2 times, about 2.3 times, about 2.4 times, about 2.5 times, about 2.6 times, about 2.7 times, about 2.8 times, about 2.9 times, about 3.0 times, about 4 times, about 5 times, or about 10 or more times larger than the number of viable cells of the wild-type cells. In one embodiment, the number of viable cells of the artificially engineered stem cells may be larger than the number of viable wild-type cells to the extent that is in the range of two numerical values selected in the previous sentence. In one embodiment, the number of viable cells of the artificially engineered stem cells may be 1.5 to 2.5 times larger than the number of viable wild-type cells.

In one embodiment, the predetermined time may be about 10 minutes, about 30 minutes, about 1 hour, about 2 hours, about 3 hours, about 4 hours, about 6 hours, about 8 hours, about 12 hours, about 24 hours, about 48 hours, about 72 hours, about 98 hours, about 120 hours, about 1 week, about 2 weeks, about 3 weeks, about 4 weeks, or about a month or more. In one embodiment, the predetermined time may be a value in the range of two numerical values selected in the immediately preceding sentence. For example, the predetermined time may be in the range of about 3 hours to about 12 hours.

### Pharmaceutically Acceptable Carrier

In one embodiment, the pharmaceutical composition for treating Alzheimer's disease disclosed herein may further include a pharmaceutically acceptable carrier in addition to the artificially engineered stem cells. In this case, the pharmaceutically acceptable carrier is a lubricant, wetting agent, sweetener, flavoring agent, emulsifying agent, suspending agent, preservative, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, mineral oil, saline, phosphate buffered saline (PBS), and/or medium, but is not limited thereto.

### Formulation of Pharmaceutical Composition

In one embodiment, the pharmaceutical composition may be formulated in a parenteral dosage form. In this case, parenteral administration includes nasal administration, eye drop administration, intravenous injection, intramuscular injection, intraperitoneal injection, transdermal administration, subcutaneous injection, intravenous injection, intramuscular injection, brain tissue injection, hippocampus injection, caudate putamen (CPu) intra-injection, intra-articular injection, intra-chondral injection, or intra-thoracic injection, etc., but is not limited thereto.

In one embodiment, in order to formulate the pharmaceutical composition into a formulation for parenteral administration, the cells as an active ingredient and a stabilizer or buffer may be mixed together with water to prepare a solution or suspension. Furthermore, the pharmaceutical composition may be formulated as ampoules or a vial unit dosage form. In one embodiment, the pharmaceutical composition may contain adjuvants such as preservatives, stabilizers, wetting agents or emulsification accelerators, salts and/or buffers for regulating osmotic pressure, and other therapeutically useful substances. The pharmaceutical composition may be formulated by mixing, granulating, or coating, which are conventional methods.

### Advantage of Pharmaceutical Composition for Treating Alzheimer's Disease

The pharmaceutical composition for the treatment of Alzheimer's disease disclosed herein is generally used in a manner of direct or indirect administration to brain tissue. When administered, the stem cells serving as an active ingredient of the pharmaceutical composition are subjected to a hypoxic environment in the brain tissue. Therefore, in the case of general stem cells, since they easily die in a hypoxic environment, it is difficult to obtain the therapeutic effect even when the stem cells are used for the treatment of Alzheimer's disease.

In comparison with this, since the engineered stem cells, which are the active ingredient of the pharmaceutical composition for treating Alzheimer's disease disclosed in the present disclosure, are characterized in that they can survive better in a hypoxic environment, they survive longer when administered to brain tissue, thereby exhibiting the intended treatment effect.

### Use of Pharmaceutical Composition

The pharmaceutical composition may be used for preventing, alleviating symptoms, and/or treating Alzheimer's disease.

### Method of Treating Alzheimer's Disease

### Overview

The present disclosure provides an Alzheimer's disease treatment method using artificially engineered stem cells or a pharmaceutical composition containing artificially engineered stem cells as an active ingredient for treating Alzheimer's disease. The Alzheimer's disease treatment method includes administering, to a subject, the artificially engineered stem cells or a pharmaceutical composition containing the artificially engineered stem cells as an active ingredient. When the pharmaceutical composition containing the artificially engineered stem cells as an active ingredient is administered to a subject, the cells can provide a significant therapeutic effect while surviving for a long time in a hypoxic environment of the administration site.

In one embodiment, the Alzheimer's disease treatment method may include administering a pharmaceutical composition containing artificially engineered stem cells as an active ingredient to a subject. In one embodiment, the subject may be a human and/or an animal. In one embodiment, the administering of the pharmaceutical composition may be performed by direct injection of the pharmaceutical composition into the brain tissue of a subject. In one embodiment, the brain tissue may be a hippocampus or a caudate putamen (CPu).

### Pharmaceutical Composition for Treating Alzheimer's Disease

The Alzheimer's disease treatment method may include administering a pharmaceutical composition containing artificially engineered stem cells as an active ingredient to a subject. In this case, the pharmaceutical composition is the same as described in the section titled "Pharmaceutical Composition for Treating Alzheimer's Disease".

### Subjects to be Administration

The Alzheimer's disease treatment method is applicable to a human and/or an animal suffering from Alzheimer's disease.

In one embodiment, the Alzheimer's disease treatment method may include administering a pharmaceutical composition containing the artificially engineered stem cells as an active ingredient to humans and/or animals. In one embodiment, the Alzheimer's disease treatment method may include administering a pharmaceutical composition containing the artificially engineered stem cells as an active ingredient to a human body. In one embodiment, the Alzheimer's disease treatment method may include administering a pharmaceutical composition containing the artificially engineered stem cells as an active ingredient to a human with Alzheimer's disease.

### Administration Site and Method

The pharmaceutical composition containing the artificially engineered stem cells as an active ingredient may be administered parenterally to a subject. In one embodiment, the Alzheimer's treatment method includes a process of administering a pharmaceutical composition containing the artificially engineered stem cells as an active ingredient to a subject by intravenous injection, intramuscular injection, intraperitoneal injection, transdermal administration, subcutaneous injection, intravenous injection, intramuscular injection, injection into brain tissue, injection into the hippocampus, and/or injection into the caudate putamen (CPu). In one embodiment, the Alzheimer's treatment method direct injection of a pharmaceutical composition containing the artificially engineered stem cells as an active ingredient into the brain tissue, hippocampus, and/or caudate putamen (CPu) of a subject.

### Dosage

In one embodiment, a suitable dosage of the pharmaceutical composition is determined by factors such as formulation method, administration method, patient's age, weight, sex, and medical condition, food, administration time, administration route, excretion rate, and response sensitivity. An ordinarily skilled practitioner can readily determine and prescribe a dosage effective for the desired treatment or prophylaxis.

### Example of Combination Treatment Method

In one embodiment, the pharmaceutical composition may be used alone or in combination with methods such as surgery, radiation therapy, hormone therapy, chemotherapy, methods of using biological response modifiers, and/or treatment methods with antibodies.

### Alzheimer's Disease Treatment Method and Result of Treatment

As a result of performing the Alzheimer's disease treatment method, the amount of amyloid beta accumulated in the subject's brain tissue may be reduced. In this case, the amyloid beta may be insoluble or soluble amyloid beta. When the Alzheimer's disease treatment method is performed, the symptoms of Alzheimer's disease of the subject can be alleviated, the symptoms of Alzheimer's disease can be improved, the progression of Alzheimer's disease can be slowed, and/or Alzheimer's disease can be treated.

### Method of Preparing Pharmaceutical Composition for Treating Alzheimer's Disease

### Overview

Hereinafter, a method of preparing a pharmaceutical composition containing the artificially engineered stem cells disclosed herein as an active ingredient will be described. The artificially engineered stem cells can be prepared by introducing a stem-cell genetic engineering composition into stem cells. In addition, by adding a pharmaceutically acceptable carrier to the artificially engineered stem cells, adding an appropriate adjuvant to the artificially engineered stem cells, and/or formulating the mixture into appropriate dosage forms or preparations, it is possible to prepare a pharmaceutical composition containing the artificially engineered stem cells as an active ingredient.

### Composition for Genetic Engineering of Stem Cells

Disclosed herein is a composition containing a CRISPR/Cas9 system that can be used to produce the artificially engineered stem cells. The CRISPR/Cas9-containing genetic engineering composition is designed to recognize and edit a suitable intracellular target sequence so that the genotype and/or phenotype of the artificially engineered stem cell can be exhibited.

In one embodiment, the genetic engineering composition may include a Cas9 protein or a DNA encoding the same, and a guide RNA or a DNA encoding the same. In this case, the Cas9 protein may be a streptococcus pyogenes-derived Cas9 protein. In this case, the sequence of the guide RNA may be a sequence selected from the group consisting of SEQ ID NOs: 22 to 30.

### Method of Preparing Artificially Engineered Stem Cells

The present disclosure discloses a method of preparing the artificially engineered stem cells. The artificially engineered stem cell preparation method includes introducing the stem-cell genetic engineering composition into stem cells.

In one embodiment, the genetic engineering method may be performed ex vivo on cells isolated from an organism in vitro. In this case, the organism may be a human or an animal, but is not limited thereto.

In one embodiment, the artificially engineered stem cell preparation method may include introducing a stem-cell genetic engineering composition into stem cells. In this case, the stem-cell genetic engineering composition may be the composition described in the section titled "Composition for Genetic Engineering of Stem Cells".

In one embodiment, the artificially engineered stem cell preparation method includes introducing a stem-cell genetic engineering composition containing ribonucleoprotein (RNP) to which Cas9 protein and guide RNA are bound into stem cells. In one embodiment, the artificially engineered stem cell preparation method includes introducing a stem-cell genetic engineering composition in which a DNA encoding a Cas9 protein and a DNA encoding a guide RNA are contained in a vector form into stem cells.

### Formulation of Pharmaceutical Composition for Treating Alzheimer's Disease into Dosage Form or Preparation - Using Existing Method

In order to prepare a pharmaceutical composition for treating Alzheimer's disease in which the pharmaceutical composition contains the artificially engineered stem cells as an active ingredient, a pharmaceutically acceptable carrier is added to the artificially engineered stem cells, an appropriate adjuvant is added, or the artificially engineered stem cells are formulated into dosage forms or preparations. In this case, the method of preparing the pharmaceutical composition by adding or adding an appropriate substance to the active ingredient and formulating the mixture appropriately is not particularly limited as long as the process does not impair the Alzheimer's disease treatment effect of the artificially engineered stem cells. In one embodiment, to prepare a pharmaceutical composition by adding a pharmaceutically acceptable carrier to the artificially engineered stem cells, adding an appropriate adjuvant to the artificially engineered stem cells, those skilled in the art may use an existing method known in the art.

### Mode for Carrying out the Invention

### Experimental Example

Hereinafter, the inventions provided by the present disclosure will be described in more detail with reference to experimental examples and examples. These examples are only for illustrating the present disclosure, and it will be apparent to those of ordinary skill in the art that the scope of the present disclosure is not to be construed as being limited by these examples.

### Experimental Example 1: Preparation of MSC with HIF1AN Knocked out

### Experimental Example 1-1: Culture of Mesenchymal Stem Cell (MSC)

As MSCs derived from human Wharton's jelly, MSCs supplied from a GMP (Good Manufacturing Practice) facility in Samsung Seoul Hospital were used. MSCs were cultured in MEM alpha 1x media (Gibco, Rockville, MD) containing 10% fetal bovine serum (FSB, Gibco) and Gentamicin 0.05 mg/mL (Thermo Fisher **Scientific) at 37°C and 5% CO₂ conditions.**

### Experimental example 1-2: Preparation of Guide RNA

RNA was transcribed in vitro, using MEGA short script T7 kit (Ambion), according to the manufacturer's instructions. A template for sgRNA was prepared through annealing and extension of two complementary oligonucleotides. The sequence of the guide domain of gRNA is shown in Table 1 below.

**[Table 1]**

| Label | Guide Domain (5' tc 3') | SEQ ID NO. | PAM | Indel (%) | Target Sequence Location (Exon) |
|---|---|---|---|---|---|
| Sp-hHIFlAN sgRNA1 | GAAGCUAUAACUGCGCAACU | 10 | GGG | 51.8 | 1 |
| Sp-hHIFlAN sgRNA2 | GGAAGCUAUAACUGCGCAAC | 11 | UGG | 9.5 | 1 |
| Sp-hHIFlAN sgRNA3 | GCAGUUAUAGCUUCCCGACU | 112 | AGG | 14.3 | 1 |
| Sp-hHIFlAN sgRNA4 | GACGCGGAAUGGGCCUAGUC | 13 | GGG | 2 | 1 |
| Sp-hHIFlAN sgRNA5 | AGACGCGGAAUGGGCCUAGU | 14 | CGG | 7.3 | 1 |
| Sp-hHIFlAN sgRNA6 | CUCUGACUCAGACGCGGAAU | 15 | GGG | 7.2 | 1 |
| Sp-hHIFlAN sgRNA7 | GGGUCGCUCUGACUCAGACG | 16 | CGG | 20.6 | 1 |
| Sp-hHIFlAN sgRNA8 | GUCUGAGUCAGAGCGACCCC | 17 | CGG | 26.5 | 1 |
| Sp-hHIFlAN sgRNA9 | CAAUAAGCUCCUCUGCCCGG | 18 | GGG | 59.8 | 1 |

The sequence of the nucleotides (crRNA repeat sequence, linker (GAAA), and tracrRNA) associated with each guide domain is SEQ ID NO: 19. The full-length sequence of the sgRNA is shown in Table 2 below.

**[Table 2]**

| Label | Guide RNA (5' to 3') | SEQ ID NO. |
|---|---|---|
| Sp-hHIFlAN sgRNA1 | GAAGCUAUAACUGCGCAACUGUUUUAGAGCUAGAAAUAGCAA GUUAAAAUAAGGCUAGUCCGUUAUCAACUUGAAAAAGUGGCA 2 CCGAGUCGGUGCUUUUUUU | 22 |
| Sp-hHIFlAN sgRNA2 | GGAAGCUAUAACUGCGCAACGUUUUAGAGCUAGAAAUAGCAA GUUAAAAUAAGGCUAGUCCGUUAUCAACUUGAAAAAGUGGCA [?] CCGAGUCGGUGCUUUUUUU | 23 |
| Sp-hHIFlAN sgRNA3 | GCAGUUAUAGCUUCCCGACUGUUUUAGAGCUAGAAAUAGCAA GUUAAAAUAAGGCUAGUCCGUUAUCAACUUGAAAAAGUGGCA 2 CCGAGUCGGUGCUUUUUUU | 24 |
| Sp-hHIFlAN sgRNA4 | GACGCGGAAUGGGCCUAGUCGUUUUAGAGCUAGAAAUAGCAA GUUAAAAUAAGGCUAGUCCGUUAUCAACUUGAAAAAGUGGCA [?] CCGAGUCGGUGCUUUUUUU | 25 |
| Sp-hHIFlAN | AGACGCGGAAUGGGCCUAGUGUUUUAGAGCUAGAAAUAGCAA | 26 |
| sgRNA5 | GUUAAAAUAAGGCUAGUCCGUUAUCAACUUGAAAAAGUGGCA CCGAGUCGGUGCUUUUUUU | |
| Sp-hHIFlAN sgRNA6 | CUCUGACUCAGACGCGGAAUGUUUUAGAGCUAGAAAUAGCAA GUUAAAAUAAGGCUAGUCCGUUAUCAACUUGAAAAAGUGGCA CCGAGUCGGUGCUUUUUUU | 27 |
| Sp-hHIFlAN sgRNA7 | GGGUCGCUCUGACUCAGACGGUUUUAGAGCUAGAAAUAGCAA GUUAAAAUAAGGCUAGUCCGUUAUCAACUUGAAAAAGUGGCA CCGAGUCGGUGCUUUUUUU | 28 |
| Sp-hHIFlAN sgRNA8 | GUCUGAGUCAGAGCGACCCCGUUUUAGAGCUAGAAAUAGCAA GUUAAAAUAAGGCUAGUCCGUUAUCAACUUGAAAAAGUGGCA CCGAGUCGGUGCUUUUUUU | 29 |
| Sp-hHIFlAN sgRNA9 | CAAUAAGCUCCUCUGCCCGGGUUUUAGAGCUAGAAAUAGCAA GUUAAAAUAAGGCUAGUCCGUUAUCAACUUGAAAAAGUGGCA CCGAGUCGGUGCUUUUUUU | 30 |

### Experimental Example 1-3: Transfection of CRISPR/Cas9 System into MSC

MSCs were transduced with the Amaxa P1 Primary Cell 4D Nucleofector kit using Program EW-104 according to the manufacturer's instructions. Specifically, Cas9 protein (4µg) premixed with ex vivo transcribed sgRNA (4µg) was incubated for 10 minutes at room temperature, and then transduced into 4 × 10⁵ cells.

### Experimental example 1-4: Targeted Deep Sequencing

Phusion polymerase (New England BioLabs) was used to amplify genomic DNA segments covering the target and potential non-target sites. The PCR amplicon products were subjected to paired-end sequencing using Illumina MiSeq.

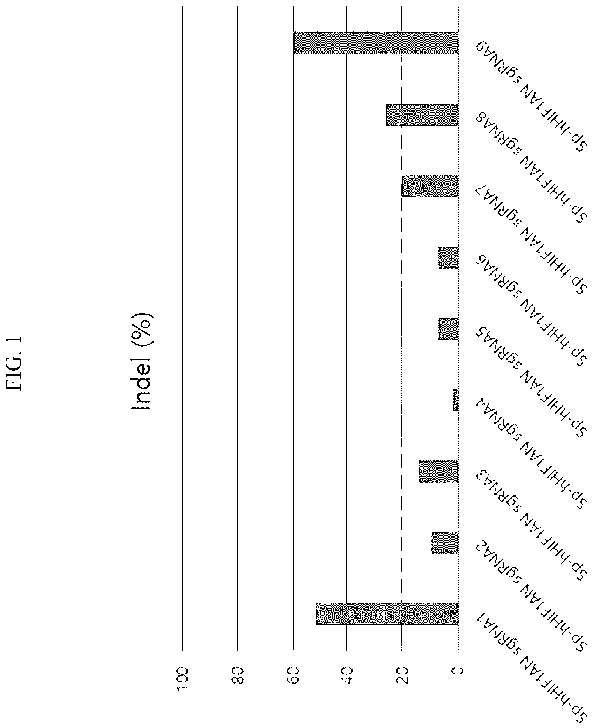

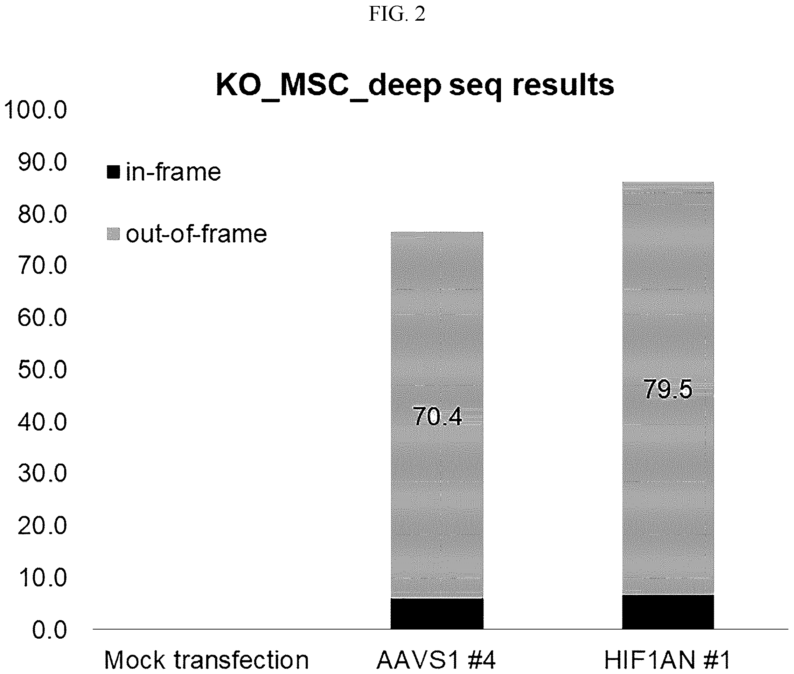

Through the results, it was confirmed that an indel was artificially introduced into the HIF1AN sequence successfully using the CRISPR/Cas9 system (see FIGS. 1 and 2 ).

### Experimental Example 2: Measurement of Viability of MSCs with HIF1AN Knocked Out

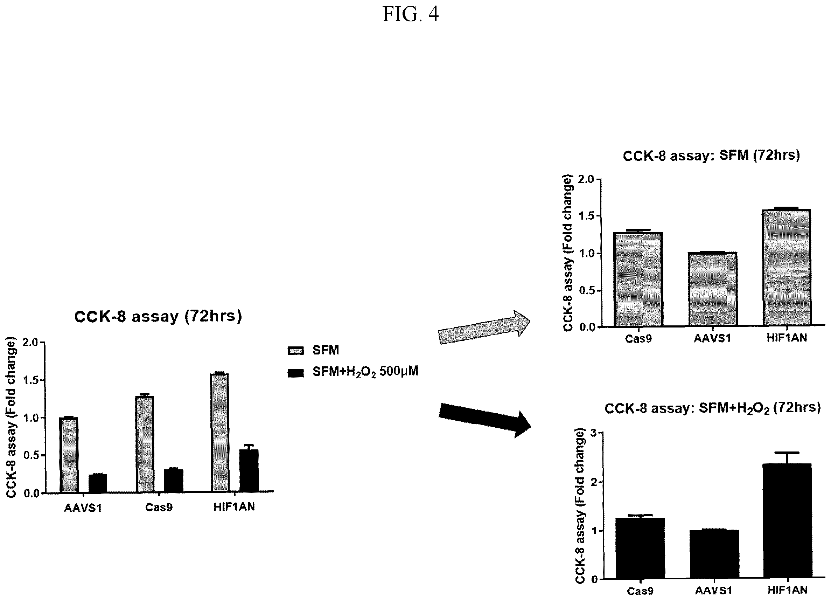

In order to investigate whether the HIF1AN-knockout MSCs obtained in Experimental Example 1 has viability, a change in the proliferative capacity of the MSCs was measured through reactive oxidative stress (ROS), which commonly occurs in a hypoxic situation. 24 hours before treating with hydrogen peroxide (H₂O₂) that induces reactive oxidative stress (ROS), 1 × 10⁴ cells were cultured in each well of a 96-well plate. After 24 hours, 500 µM of peroxide (H₂O₂) was added to a serum-free medium and incubated. In order to check the cell viability in 72 hours after the H₂O₂ treatment, the cell viability was measured with a CCK-8 cell proliferation kit (Dojindo^{®}, Japan) (see FIG 4). As a result, it was confirmed that the survival rate of MSCs in which the HIF1AN was knocked out in an environment treated with hydrogen peroxide (H₂O₂), which induces reactive oxidative stress (ROS), was improved than that of the control group.

### Experimental Example 3: Alzheimer's Treatment Effect of HIF1AN-Knockout (K/O) MSC

### Experimental example 3-1: Administration of MSC to Normal Mice

After MSC administration into the mouse brain, an experiment was performed to determine the difference in the remaining stem cells in the brain between the control group and the HIF1AN K/O stem cells. As an animal model, normal mice (C57BL/6, 7-8 weeks old) were used. First, 2 × 10⁵ cells (2 × 10⁵/5 µL) of human-derived mesenchymal stem cells were administered to the hippocampus of normal mice. After setting the zero point of the coordinates based on bregma (A/P -2.06 mm, M/L -1.3 mm, and D/V -2.0 mm) by using a stereotaxic device (Harvard apparatus, USA), the HIF1AN K/O stem cells and the control were administered to the left hippocampus at a flow rate of 0.5 µL/min. As controls, naive MSCs which are stem cells without having undergone gene editing, and AAVSI MSCs which are stem cells having undergone gene editing and including no target genes were administered.

### Experimental example 3-2: Measurement of Remaining Cells of Stem Cells Administered to Normal Mice

One week after the administration, normal mice were euthanized by coronary perfusion, and brains were removed. After the extracted brain tissue was disrupted using a homogenizer, genomic DNA was extracted using the Gentra puregene kit (QIAGEN, USA). To check the residual amount of human-derived stem cells in mouse brain tissue, real-time PCR was performed using an ALU element capable of detecting a human-specific DNA sequence. Primer information is as follows: ALU forward: 5' - CAT GGT GAA ACC CCG TCT CTA - 3' (SEQ ID NO: 31), ALU reverse: 5' - GCC TCA GCC TCC CGA GTA G -3' (SEQ ID NO: 32). 20 ng of each sample DNA was added to a mixture of a primer and a SYBR green master mix (Life Technologies, USA), and real-time PCR was performed. The PCR conditions were as follows: a total of 40 cycles, 95°C (10 min), 95°C (15 sec), 68°C (30 sec), and 72°C (30 sec).

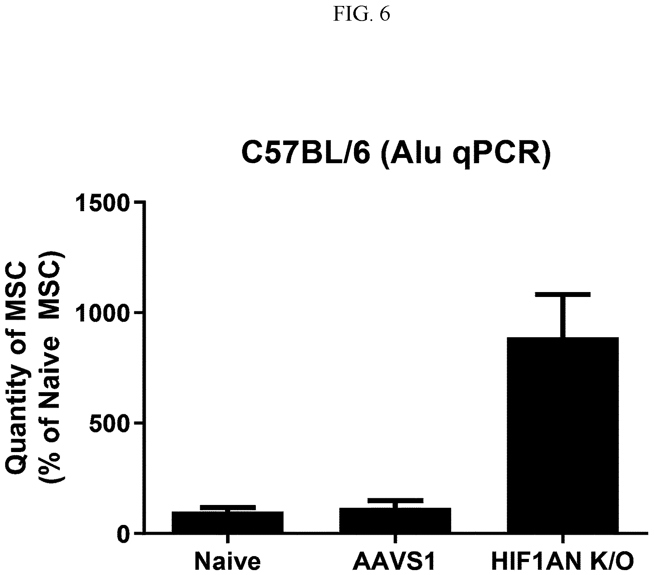

The results showed that the residual amount of the HIF1AN K/O stem cells was 8.9 and 7.6 times higher than that of the naive and AAVS1 controls, respectively (FIG. 6).

### Experimental example 3-3: Administration of MSCs to Mice with Alzheimer's Disease

After MSC administration into mouse brain, an experiment was performed to determine the difference in the remaining stem cells in the brain between the control group and the HIF1AN K/O stem cells. As an animal model, Alzheimer's disease mice (5xFAD, 7-8 months old) were used. First, 2 × 10⁵ cells (2 × 10⁵/5 µL) of human-derived mesenchymal stem cells were administered to the hippocampus of 5xFAD, which are mice with Alzheimer's disease. After setting the zero point of the coordinates based on bregma (A/P -2.06 mm, M/L ± 1.3 mm, and D/V -2.0 mm) by using a stereotaxic device (Harvard apparatus, USA), the HIF1AN K/O stem cells and the controls were administered to the right hippocampus at a flow rate of 0.5 µL/min. As the controls, naive MSCs which are stem cells without having undergone gene editing, and AAVSI MSCs which are stem cells having undergone gene editing and including no target genes were administered.

### Experimental example 3-4: Measurement of Remaining Cells of Stem Cells Administered to Mice with Alzheimer's disease

When two weeks passed after the administration, 5xFAD mice were euthanized by coronary perfusion, and brains were removed. After the extracted brain tissue was disrupted using a homogenizer, genomic DNA was extracted using the Gentra puregene kit (QIAGEN, USA). To check the residual amount of human-derived stem cells in mouse brain tissue, real-time PCR was performed using an ALU element capable of detecting a human-specific DNA sequence. Primer information is as follows: ALU forward: 5' - CAT GGT GAA ACC CCG TCT CTA - 3' (SEQ ID NO: 31), ALU reverse: 5' - GCC TCA GCC TCC CGA GTA G -3' (SEQ ID NO: 32). 20 ng of each sample DNA was added to a mixture of a primer and a SYBR green master mix (Life Technologies, USA), and real-time PCR was performed. The PCR conditions were as follows: a total of 40 cycles, 95°C (10 min), 95°C (15 sec), 68°C (30 sec), and 72°C (30 sec) .

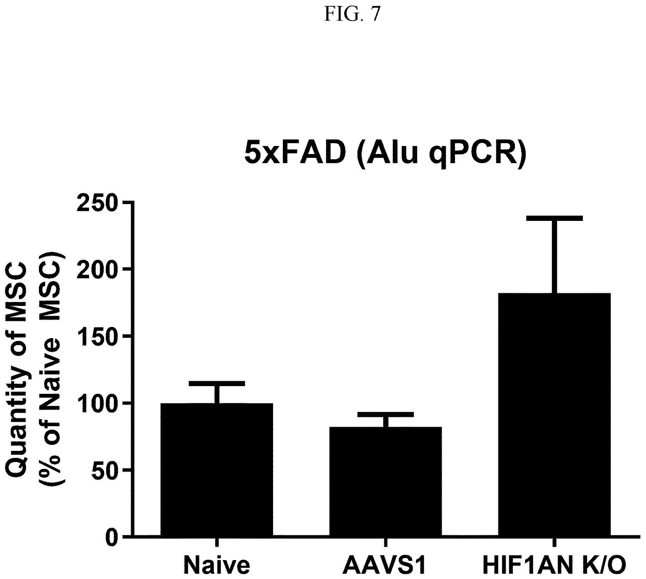

The results showed that the residual amount of the HIF1AN K/O stem cells was larger than that of the naive and AAVS1 controls (FIG. 7).

### Experimental Example 3-5: Analysis of Therapeutic Effect of HIF1AN K/O Stem Cells in Alzheimer's Disease Mouse Model

Stem cells were administered to the hippocampus of an Alzheimer's disease mouse in the same manner as in Experimental Example 3-3. 2 weeks later, the Alzheimer's disease mouse was euthanized by coronary perfusion, and the brain thereof was extracted. The extracted brain tissue was crushed using a medicine bowl, and then soluble protein was extracted. To extract the soluble protein, the crushed brain tissue was dissolved in a homogenization buffer (20 mM Tris pH 7.4, 250 mM sucrose, 1 mM EDTA, 1 mM EGTA, 1X protease inhibitor cocktail) and centrifuged at 10,000 g at 4°C for 1 hour. The supernatant was separated and used for detection of soluble proteins. A β 42 ELISA Kit (Thermo Scientific, USA) was used to determine the difference in the expression of amyloid protein between samples. To check whether Soluble A β was expressed, 2 µg of each sample was used.

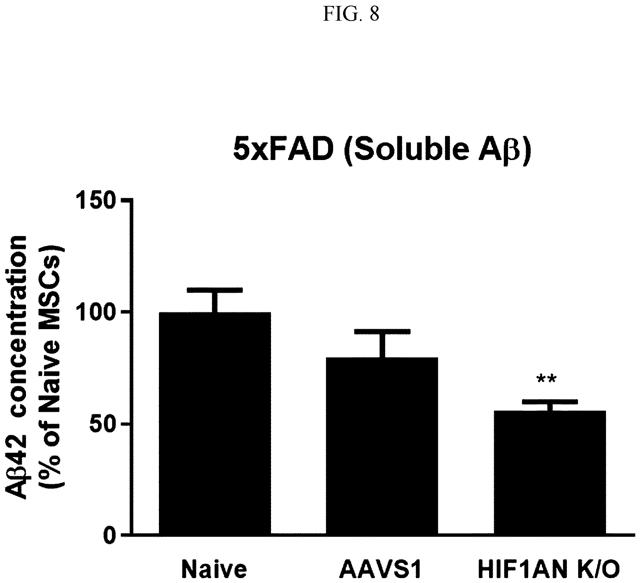

As a result, soluble A β expression was significantly reduced in the group administered with HIF1AN K/O stem cells compared to the controls (naive and AAVS1) (see FIG. 8).

From these results, it was confirmed that the HIF1AN K/O stem cells showed improved viability under hypoxic environment. When administered to brain tissue, it was confirmed that the amount of amyloid beta was significantly reduced, indicating the therapeutic effect to Alzheimer's disease. Since there is no proper cell therapy for Alzheimer's disease, the invention disclosed in the present application will be useful in the art as a symptom relief or therapeutic agent for Alzheimer's disease.

### Industrial Applicability

The present disclosure can provide a stable and effective medicament or therapeutic pharmaceutical composition for treating Alzheimer's disease and also can provide an Alzheimer's disease treatment method using the same.

## Claims

1. A pharmaceutical composition comprising an artificially manipulated stem cell as an active ingredient for treating the Alzheimer's disease, the manipulated stem cell comprising an engineered gene having an indel in a wild-type HIF1AN gene,
wherein a sequence of the engineered HIF1AN gene is different from the original sequence of the wild-type HIF1AN gene,
FIH-1 exhibits a lower expression level in the manipulated stem cell than in the wild-type cell, and
HIFα exhibits a higher expression level in the manipulated stem cell than in the wild-type cell.

2. The pharmaceutical composition of claim 1, wherein the indel of the engineered HIF1AN gene is located in an exon 1 region of the wild-type HIF1AN gene.

3. The pharmaceutical composition of claim 1, wherein the manipulated stem cell is an engineered mesenchymal stem cell.

4. The pharmaceutical composition of claim 1, wherein the pharmaceutical composition has a formulation for administration to brain tissue.

5. The pharmaceutical composition of claim 4, wherein the brain tissue is a hippocampus or a caudate putamen (CPu).

6. A pharmaceutical composition comprising an manipulated stem cell as an active ingredient for treating the Alzheimer's disease, the manipulated stem cell comprising an engineered HIF1AN gene,
wherein the engineered HIF1AN gene does not comprise one or more sequences selected from SEQ ID NOs:1 to 9,
FIH-1 exhibits a lower expression level in the manipulated stem cell than in the wild-type cell, and
HIFα exhibits a higher expression level in the manipulated stem cell than in the wild-type cell.

7. A method of treating Alzheimer's disease, the method comprising: administering a pharmaceutical composition comprising an manipulated stem cell as an active ingredient into a subject,
wherein the manipulated stem cell comprises an engineered gene having an indel in a wild-type HIF1AN gene,
a sequence of the engineered HIF1AN gene is different from the original sequence of the wild-type HIF1AN gene,
FIH-1 exhibits a lower expression level in the manipulated stem cell than in the wild-type cell, and
HIFα exhibits a higher expression level in the manipulated stem cell than in the wild-type cell.

8. The method of claim 7, wherein the administration of the pharmaceutical composition is a direct injection of the pharmaceutical composition to a brain tissue of the subject.

9. The method of claim 8, wherein the brain tissue is a hippocampus or a caudate putamen (CPu).

10. The method of claim 7, wherein the indel of the engineered HIF1AN gene is located in an exon 1 region of the wild-type HIF1AN gene.

11. The method of claim 7, wherein the manipulated stem cell is an engineered mesenchymal stem cell.

12. A method of treating Alzheimer's disease, the method comprising: administering a pharmaceutical composition comprising an manipulated stem cell as an active ingredient into a subject,
wherein the manipulated stem cell comprises an engineered HIF1AN gene,
the engineered HIF1AN gene does not comprise one or more sequences selected from SEQ ID NOs:1 to 9,
FIH-1 exhibits a lower expression level in the manipulated stem cell than in the wild-type cell, and
HIFα exhibits a higher expression level in the manipulated stem cell than in the wild-type cell.

13. A use of an manipulated stem cell for treating Alzheimer's disease,
wherein the use comprises administering a pharmaceutical composition comprising an manipulated stem cell as an active ingredient into a subject,
the manipulated stem cell comprises an engineered gene having an indel in a wild-type HIF1AN gene,
a sequence of the engineered HIF1AN gene is different from the original sequence of the wild-type HIF1AN gene,
FIH-1 exhibits a lower expression level in the manipulated stem cell than in the wild-type cell, and
HIFα exhibits a higher expression level in the manipulated stem cell than in the wild-type cell.

14. The use of claim 13, wherein the administration of the pharmaceutical composition is a direct injection of the pharmaceutical composition to a brain tissue of the subject.

15. The use of claim 14, wherein the brain tissue is a hippocampus or a caudate putamen (CPu).

16. The use of claim 13, wherein as to the engineered HIF1AN gene, the indel of the wild-type HIF1AN gene is located in an exon 1 region of the wild-type HIF1AN gene.

17. The use of claim 17, wherein the manipulated stem cell is an engineered mesenchymal stem cell.

18. A use of an manipulated stem cell for treating Alzheimer's disease,
wherein the use comprises administering a pharmaceutical composition comprising an manipulated stem cell as an active ingredient into a subject,
the manipulated stem cell comprises an engineered HIF1AN gene,
the engineered HIF1AN gene does not comprise one or more sequences selected from SEQ ID NOs:1 to 9,
FIH-1 exhibits a lower expression level in the manipulated stem cell than in the wild-type cell, and
HIFα exhibits a higher expression level in the manipulated stem cell than in the wild-type cell.

19. A use of an manipulated stem cell for the manufacture of a medicament for treatment of Alzheimer's disease,
wherein the manipulated stem cell comprises: an engineered gene having an indel in a wild-type HIF1AN gene,
wherein a sequence of the engineered HIF1AN gene is different from the original sequence of the wild-type HIF1AN gene,
FIH-1 exhibits a lower expression level in the manipulated stem cell than in the wild-type cell, and
HIFα exhibits a higher expression level in the manipulated stem cell than in the wild-type cell.

20. The use of claim 19, wherein the pharmaceutical composition is a formulation for administration to a brain tissue.

21. The use of claim 20, wherein the brain tissue is a hippocampus or a caudate putamen (CPu).

22. The use of claim 19, wherein the indel of the wild-type HIF1AN gene is located in an exon 1 region of the wild-type HIF1AN gene.

23. The use of claim 19, wherein the manipulated stem cell is an engineered mesenchymal stem cell.

24. A use of an manipulated stem cell for the manufacture of a medicament for treatment of Alzheimer's disease,
wherein the use comprises administering a pharmaceutical composition comprising an manipulated stem cell as an active ingredient into a subject,
wherein the manipulated stem cell comprises an engineered HIF1AN gene,
the engineered HIF1AN gene does not comprise one or more sequences selected from SEQ ID NOs:1 to 9,
FIH-1 exhibits a lower expression level in the manipulated stem cell than in the wild-type cell, and
HIFα exhibits a higher expression level in the manipulated stem cell than in the wild-type cell.
